# EUROPEAN PATENT SPECIFICATION

(11) **EP 2 491 402 B1**
(45) Date of publication and mention of the grant of the patent: **21.08.2019**
(21) Application number: 10769084.4
(22) Date of filing: 19.10.2010
(51) Int. Cl.: G01N 33/86

(54) **BLOOD COAGULATION STATUS MEASUREMENT IN SALIVA**
MESSUNG DES BLUTGERINNUNGSSTATUS IM SPEICHEL
MESURE DU STATUT DE COAGULATION SANGUINE DANS LA SALIVE

(30) Priority: 19.10.2009 WO PCT/NL2009/050629
(43) Date of publication of application: 29.08.2012
(73) Proprietor: Stichting Katholieke Universiteit, Radboud Universiteit Nijmegen, 6525 HP Nijmegen (NL)
(72) Inventor: LOOF, Arnoud Harmen, NL-6663 HV Lent (NL); VAN HEERDE, Waander Laurens, NL-6531 JS Nijmegen (NL)
(74) Representative: Nederlandsch Octrooibureau
(86) International application number: PCT/NL2010/050693
(87) International publication number: WO 2011/049442

(56) References cited:
- WO-A1-01/63299
- WO-A1-99/60409
- TIBOR K FABIAN ET AL: "Saliva in health and disease, chemical biology of", 1 January 2007 (2007-01-01), WILEY ENCYCLOPEDIA OF CHEMICAL BIOLOGY,, PAGE(S) 1 - 9, XP009128562, pages 5,6; table 2
- NOUR-ELDIN F ET AL: "The blood clotting factors in human saliva", 30 April 1957 (1957-04-30), JOURNAL OF PHYSIOLOGY, WILEY-BLACKWELL PUBLISHING LTD, GB, PAGE(S) 324 - 332, XP009128369, ISSN: 0022-3751 the whole document
- KAUFMAN ELIAZ ET AL: "The diagnostic applications of saliva--a review", 1 January 2002 (2002-01-01), CRITICAL REVIEWS IN ORAL BIOLOGY AND MEDICINE, CRC PRESS, BOCA RATON, FL, US, PAGE(S) 197 - 212, XP009119571, ISSN: 1045-4411 the whole document
- KUZNIK B I ET AL: "Secretion of blood coagulation factors into saliva under conditions of hypo- and hypercoagulation", 1973, VOPROSY MEDITSINSKOJ KHIMII 1973, VOL. 19, NR. 1, PAGE(S) 54 - 57, XP009128371, ISSN: 0042-8809 abstract
- ZACHARSKI L R ET AL: "Reduction of salivary tissue factor (thromboplastin) activity by warfarin therapy", 1 March 1979 (1979-03-01), BLOOD, AMERICAN SOCIETY OF HEMATOLOGY, US, PAGE(S) 366 - 374, XP009042344, ISSN: 0006-4971 abstract
- FÁBIÁN T K ET AL: "Salivary Genomics, Transcriptomics and Proteomics: The Emerging Concept of the Oral Ecosystem and their Use in the Early Diagnosis of Cancer and other Diseases.", March 2008 (2008-03), CURRENT GENOMICS MAR 2008, VOL. 9, NR. 1, PAGE(S) 11 - 21, XP002565375, ISSN: 1389-2029 abstract
- I. Iontcheva ET AL: "Human Salivary Mucin MG1 Selectively Forms Heterotypic Complexes with Amylase, Proline-rich Proteins, Statherin, and Histatins", JOURNAL OF DENTAL RESEARCH, vol. 76, no. 3, 1 March 1997 (1997-03-01), pages 734-743, XP055326010, US ISSN: 0022-0345, DOI: 10.1177/00220345970760030501

## Description

### Field of the invention

The present invention is in the field of medicine, in particular in the field of blood coagulation. More specifically, the invention relates to a method for measuring the blood (anti)coagulation status, e.g., the international normalized ratio (INR), in saliva rather than blood samples.

### Background

Vitamin K serves as an essential cofactor for a carboxylase that catalyses carboxylation of glutamic acid residues on vitamin K-dependent proteins. The key vitamin K-dependent proteins include coagulation proteins such as factors II (prothrombin), VII, IX and X, and anticoagulation proteins C, S and Z. These proteins have in common the requirement to be post-translationally modified by carboxylation of glutamic acid residues (forming gamma-carboxyglutamic acid) in order to become biologically active. Prothrombin, for example, has 10 glutamic acids in the amino-terminal region of the protein, which are carboxylated. Without vitamin K, the carboxylation does not occur and the proteins that are synthesized are biologically inactive.

Vitamin K antagonists (VKA) are world wide the most frequently described anticoagulants. The use of VKAs in routine clinical practice presents a number of challenges: a complex dose-response relationship, susceptibility to interactions with numerous foods, drugs and dietary supplements, genetic influences, and concomitant disease. In addition, VKAs have a narrow therapeutic window which necessitates frequent dose adjustments to maintain the international normalized ratio (INR) dependent on the indication. The INR measures the time it takes for blood to clot and compares it to an average. Monitoring the INR is considered an important step in managing health. To find the INR, routinely a small sample of blood is taken from a fingertip or vein. The time it takes the blood to clot is measured. Then a ratio (the INR) is established. The higher the INR, the longer it takes blood to clot. If the patient receives too little anticoagulation then protection against thrombotic events is inadequate, too much and the patient's risk for major hemorrhage increases. These factors impose considerable demands on both patients and physicians, and oral anticoagulation therapy with VKAs remains a significant challenge in routine clinical practice. As a result, many patients are either untreated or receive inadequate anticoagulation therapy, according to treatment guideline.

Currently, routine patient testing of the INR is performed using clotting assays, either by a conventional laboratory, or in specialty anticoagulation clinics, with results being interpreted and managed by a physician. Moreover, INR can also be monitored by point of care devices, but the interpretation and management is concentrated by a physician. Clotting assays present several drawbacks. Most importantly, they are by definition invasive, as they require blood to be drawn from a patient.

Saliva, because of its easy, non-invasive mode of collection, would be an attractive diagnostic fluid to overcome at least one of the drawbacks of clotting assays. Saliva is a secretion of the salivary and mucous glands and is of major importance in the maintenance of oral health. A subject can nearly always salivate at any time of day, privacy is not required to provide a saliva sample, and collection of a sample is fast. However, it is as yet unknown how proteins in saliva correlate to the blood coagulation status of a subject.

US 6,503,724 discloses that fibrinopeptides in saliva may be used as indicator of blood coagulation status. Prothrombin fragment 1+2 (F1+2), prothrombin fragment 1 (F1), prothrombin fragment 2 (F2), fibrinopeptide A (FpA) and D-Dimers in saliva were quantitatively measured using an enzyme immunoassay and correlated with the coagulation time as expressed for example by prothrombin time (PT), INR, partial thromboplastin time (PTT) and activated partial thromboplastin time (APTT).

In the present study whole stimulated saliva of 16 patients with anticoagulation therapy were compared to 16 healthy controls matched for age and sex to check for the presence of proteins present in saliva that correlate with the anticoagulation status. The present inventors have used surface-enhanced laser desorption ionization time-of-flight mass spectrometer (SELDI-TOF-MS) to look for differentially expressed proteins in saliva of the 2 subject populations. The inventors identified several proteins that were differentially expressed in the 2 populations. Subsequently, the levels of some of these differentially expressed proteins were correlated to the INR to identify those proteins whose level was strongly correlated to the INR.

### Summary of the Invention

The present disclosure relates to a method for determining the blood coagulation status of a subject, said method comprising the steps of: a) providing a sample of saliva of said subject; b) determining the level of one or more biomarkers in said sample of saliva; and c) correlating the amount of said one or more biomarkers to the blood coagulation status.

The present invention relates to a method for determining the blood coagulation status of a subject, said method comprising the steps of: a) providing a sample of saliva of said subject; b) determining the level of one or more biomarkers in said sample of saliva; and c) correlating the amount of said one or more biomarkers to the blood coagulation status wherein one of said biomarker is statherin.

In a further aspect, the present disclosure relates to a kit suitable for performing the method according to any of the preceding claims comprising one or several separate sealed containers comprising at least one antibody with specificity for one or more biomarkers, said biomarkers being selected from the group of biomarkers having a molecular mass as determined by SELDI-TOF-MS as present in Tables 1, 2 or 3, and an enzymatic substrate to produce a visible signal.

In a further aspect, the present disclosure relates to a kit suitable for performing the method according to any of the preceding claims comprising one or several separate sealed containers comprising at least one antibody with specificity for one or more biomarkers, said biomarkers being statherin, and an enzymatic substrate to produce a visible signal.

In another aspect, the present disclosure is concerned with a method for identifying one or more biomarkers correlating with blood coagulation status in a subject, said method comprising the step of: i) subjecting a saliva sample from said subject to mass spectrometry; ii) correlating the peaks/molecular masses in the mass spectrum obtained in i) with blood coagulation status of said subject; and iii) determining peptide identity of the blood coagulation status correlated peaks in the mass spectrum.

### Detailed Description of Embodiments

The present inventors compared whole stimulated saliva of 16 patients with anticoagulation therapy to 16 healthy controls matched for age and sex to check for the presence of proteins present in saliva that correlate with the anticoagulation status. The present inventors used surface-enhanced laser desorption ionization time-of-flight mass spectrometer (SELDI-TOF-MS) to look for differentially expressed proteins in saliva of the 2 subject populations. Masses (m/z)-values obtained from these SELDI-TOF-MS analyses were searched against protein databases.

The inventors identified several proteins that were differentially expressed in the 2 populations. Subsequently, the concentrations of some of these differentially expressed proteins were correlated to the INR to identify those proteins whose concentration was strongly correlated to the INR. In this way, the concentration of those proteins in saliva may be used as an indicator of the blood coagulation status (e.g., INR) of a subject.

In a first aspect, the present invention relates to a method for determining the blood coagulation status of a subject, said method comprising the steps of: a) providing a sample of saliva of said subject; b) determining the level of one or more biomarkers in said sample of saliva; and c) correlating the amount of said one or more biomarkers to the blood coagulation status, wherein one of said biomarkers is statherin.

In a first aspect, the present disclosure relates to a method for determining the blood coagulation status of a subject, said method comprising the steps of: a) providing a sample of saliva of said subject; b) determining the level of one or more biomarkers in said sample of saliva; and c) correlating the amount of said one or more biomarkers to the blood coagulation status.

The blood coagulation status of a subject treated with VKA's may be expressed by prothrombin time (PT), international normalized ratio (INR), or any other means known in the art. Preferably, the blood coagulation status of said subject is expressed as INR.

The term "International Normalized Ratio" ("INR") is well known by the skilled person. The INR was created to standardize prothrombin time (PT) measurements in various laboratories using various types (brands) of reagents. The various reagents are compared to a standard from the World Health Organisation (WHO) and are awarded an International Sensitivity Index (ISI) that indicates their sensitivity compared to the WHO standard. To this end, the PT of several patients is determined with both the WHO standard (y-axis) and the reagent that is being investigated (x-axis). A straight line can be drawn between the points on the curve. Based on this index the local PT is calculated. Thus, the INR is the (PTₚₐₜᵢₑₙₜ/PTₙₒᵣₘₐₗ)^{ISI}.

The term "saliva" as used herein includes saliva, filtered saliva, and sputum. The invention is applicable to both humans and non-human animals. The method is by its very nature non-invasive. The method of the invention may preferably be carried out on saliva obtained from mammalian subjects, most preferably on human subjects although the method may also be adapted for use in various animals, bovine, canines, cats, horses, sheep, goats, pigs and rodents. The method of the invention may be readily carried out on whole saliva, but may preferably be carried out on parotid saliva or stimulated whole saliva that is rich in parotid saliva, to obtain optimal results for determining biomarkers for blood coagulation status.

The method of the invention comprises the detection of one or more biomarkers that may increase or decrease in response to the blood coagulation status. Preferably, the method of the invention comprises the sequential or simultaneous detection of 2, 3, 4, 5, 6, 7, or more biomarkers that increase or decrease with changes in the blood coagulation status in the subject, in order to increase the sensitivity and/or consistency/reproducibility of the method. Most preferably, the method of the invention comprises the detection of 2 to 6 biomarkers of blood coagulation status. Preferably, at least one of these biomarkers is up-regulated and at least one of these biomarkers is down-regulated. The biomarkers of the invention that correlate with blood coagulation status may correlate positively or negatively, i.e. may increase or decrease in saliva depending on the blood coagulation status.

The change in the level of blood coagulation biomarkers in saliva may be determined as the fold increase/decrease, or alternatively as the relative increase or decrease of protein in the saliva of a subject in which blood coagulation status may be compromised as compared to protein in the saliva sample of a healthy subject. Biomarkers of blood coagulation status may also be measured as increases/decreases in absolute level (i.e., concentration or amount) in saliva.

In the method of the invention, the level of said one or more proteins may be determined by methods well known in the art, e.g., using an enzyme immunoassay or mass spectrometry, e.g., by obtaining a Surface-Enhanced Laser Desorption/Ionization Time-Of-Flight Mass Spectrometry (SELDI-TOF-MS) spectrum of the sample or using other mass spectrometric techniques.

The term "enzyme immunoassay" ("EIA"), also called enzyme-linked immunosorbent assay (ELISA), is a biochemical technique that is well known in the art. It is used mainly in immunology to detect the presence of an antibody or an antigen in a sample. In ELISA, an unknown amount of antigen is affixed to a surface, and then a specific antibody is washed over the surface so that it can bind to the antigen. This antibody is linked to an enzyme, and in the final step a substance is added that the enzyme can convert to some detectable signal. Thus in the case of fluorescence ELISA, when light of the appropriate wavelength is shone upon the sample, any antigen/antibody complexes will fluorescence so that the amount of antigen in the sample can be inferred through the magnitude of the fluorescence signal.

Performing an ELISA involves at least one antibody or other binding partners with high affinity for the biomarker with specificity for a particular antigen. The sample with an unknown amount of antigen may be immobilized on a solid support (for example, a polystyrene microtiter plate) either non-specifically (via adsorption to the surface) or specifically (via capture by another antibody specific to the same antigen, in a "sandwich" ELISA). After the antigen is immobilized the detection antibody is added, forming a complex with the antigen. The detection antibody can be covalently linked to an enzyme, or can itself be detected by a secondary antibody which is linked to an enzyme through bioconjugation. Between each step the plate is typically washed with a mild detergent solution to remove any proteins or antibodies that are not specifically bound. After the final wash step the plate is developed by adding an enzymatic substrate to produce a visible signal, which indicates the quantity of antigen in the sample. The substrates may utilize chromogenic substrates, though fluorogenic substrates and chemo luminescent substrates are used more commonly as they enable higher sensitivity. For some of the biomarkers of the invention, monoclonal antibodies may be commercially available from various suppliers all of which may be successfully applied, taking into account the manufacturers recommendations for use.

Alternatively, the amount of protein may be determined using other routine techniques, including, but not limited to, capillary action, precipitation, turbidimetric, diffusion, agglutination, potentiometric, amperometric, piezoelectric and evanescent-wave immunosensors, or any combination thereof.

In an embodiment of the disclosure, the biomarkers are selected from the group of biomarkers having a molecular mass as determined by SELDI-TOF-MS as present in Tables 1, 2 or 3. These correlate with blood coagulation status (compare healthy subjects to patients).

For the purposes of the present invention, biomarkers having a molecular mass deviating (+/-) from the exact molecular masses presented in Tables 1, 2, or 3 by at most 2.5%, such as at most 2%, 1.75%, 1.5%, 1.25%, 1%, 0.9%, 0.8%, 0.7%, 0.6%, or 0.5% are also included in the term "biomarkers having a molecular mass as determined by SELDI-TOF-MS as present in Tables 1, 2, or 3". In a preferred embodiment, the biomarkers having a molecular mass as determined by SELDI-TOF-MS as present in Tables 1, 2, or 3 deviate from the exact molecular masses mentioned by at most 0.5%, such as at most 0.4%, 0.3%, 0.2%, 0.1%, 0.09%, 0.08%, 0.07%, 0.06%, or 0.05%.

In a further embodiment of the disclosure, the one or more biomarkers are selected from the group consisting of BNP-32; S100A8; acidic salivary proline-rich protein 1/2; statherin; α-haemoglobin; acidic salivary proline-rich protein 3/4; S100A9, and thymosin beta-4. The identity of these proteins of Tables 1 and 2 has been confirmed by peptide mapping or sequenced by high resolution ms-instruments, or have been measured using an enzyme immunoassay.

In an embodiment of the disclosure, the one or more biomarkers are selected from the group consisting of BNP-32; S1008A; and acidic salivary proline-rich protein 1/2. These proteins seem to have the highest correlation with INR.

In a further embodiment, the blood coagulation status is determined by the international normalized ratio (INR).

An interesting application of this invention would be to control the level of any compound, composition, or pharmaceutical which results in anticoagulation of the blood. The method of the invention may also be applied for screening compounds, compositions, or pharmaceuticals for their capability to increase or reduce blood coagulation status in a subject. The method allows for an easy, cost-effective and rapid screening of blood coagulation status in a subject without having to collect blood samples at multiple time points, which is unpleasant for the subject, more laborious and expensive. Additionally, blood sampling is known to cause a mild stress response in certain individuals. A stress response may influence biomarkers under study, which is highly disadvantageous and may be circumvented using the method and means provided by the present invention.

In an embodiment, the blood coagulation status is related to the intake of anticoagulant or procoagulant drugs.

In an embodiment, the subject is undergoing VKA therapy, or other anticoagulant or procoagulant therapy.

In a further embodiment, the blood coagulation status is related to a blood coagulation disease or disorder. The blood coagulation disease or disorder may be an acute thrombotic situation, such as acute myocardial infarction or pulmonary embolism or acute hemorrhages.

The use of proteins in saliva as an indicator of blood coagulation status might also allow for easy-to-use self-testing systems of such blood coagulation status These devices allow more frequent monitoring to assess time in therapeutic range and to detect any drift in blood coagulation status, e.g., INR, prior to a clinically significant event. Patients may then receive anticoagulant dose adjustments from their physicians, or may adjust their own dose.

Thus, the disclosure further pertains to a non-invasive device for performing *in vitro* analysis of the level of the coagulation markers which best correlate to the state of anticoagulation of subjects undergoing anticoagulation therapy or other therapy related to coagulation (such as coagulation suppletion therapy) so that the subjects can control their dosage, thereby reducing visits to conventional laboratories and specialty anticoagulation clinics and increasing the accuracy of dosing. The functional requirements of the test are: be non-invasive, no need to draw a blood sample from a user, capable of being used at home by persons undergoing anticoagulation therapy or other therapy related to coagulation (such as coagulation suppletion therapy), capable of being carried out by subjects of average intelligence and motor skills after minimal training, results should be readable by the user's eye or an inexpensive device such as a photometer, densitometer or calorimeter, should be cost-effective (costs lower than the costs charged by physicians and clinics for the patient to visit and carry out the test procedure),capable of being accurately measured for a subject stabilized on anticoagulation therapy, sample acquisition and processing should be easy, portable, rapid results required, quantitative result required.

Such a point of care or bedside test would save time and money, and helps those subjects for which access to the clinics is difficult. A point of care or bedside test would also improve anticoagulation therapy and safety as self-monitoring has proven more accurate than institution-based monitoring. Also, subjects prefer to self-control rather than spend their time visiting a clinic.

In a further aspect, the invention is concerned with a kit suitable for performing the method according to any of the preceding claims comprising one or several separate sealed containers comprising at least one antibody with specificity for one or more biomarkers, wherein one of said biomarkers is statherin.

In a further aspect, the disclosure is concerned with a kit suitable for performing the method according to any of the preceding claims comprising one or several separate sealed containers comprising at least one antibody with specificity for one or more biomarkers, said biomarkers being selected from the group of biomarkers having a molecular mass as determined by SELDI-TOF-MS as present in Tables 1, 2 or 3, and an enzymatic substrate to produce a visible signal.

In an embodiment of the disclosure, the one or more biomarkers are selected from the group consisting of BNP-32; S100A8; acidic salivary proline-rich protein 1/2; statherin; α-haemoglobin; and acidic salivary proline-rich protein 3/4; S100A9, and thymosin beta-4.

An antibody with specificity for one of the biomarkers mentioned herein may be prepared by methods known to the skilled person. For example, such protein or an immunogenic fragment thereof may be injected into a New Zealand White rabbit. The rabbit may then produce an antibody which may be a polyclonal antibody. The antibody may subsequently be purified by methods which are routinely used in the art, and well known to the skilled person.

The enzymatic substrate may be any substrate providing a signal which can be observed, but preferably is a fluorogenic or chemiluminescent substrate.

In a further aspect, the present invention pertains to a method for identifying one or more biomarkers correlating with blood coagulation status in a subject, said method comprising the step of: i) subjecting a saliva sample from said subject to mass spectrometry; ii) correlating the peaks/molecular masses in the mass spectrum obtained in i) with blood coagulation status of said subject; and iii) determining peptide identity of the blood coagulation status correlated peaks in the mass spectrum. Preferably, the method comprises the sequential detection of biomarker levels in saliva obtained from a healthy subject and a subject in which blood coagulation status is compromised. The two different conditions and comparison of the two obtained mass spectra from saliva samples may for example reflect the biomarker spectrum of a patient undergoing anticoagulant or procoagulant therapy compared to a healthy subject. The biomarkers and/or peptides identified by this method that correlate either positively or negatively with blood coagulation status, may be suitably applied in the method of the invention for determining the blood coagulation status of a subject. In addition, the identified biomarkers may be applied to prepare antibodies or functional fragments thereof that are specific for the biomarker of blood coagulation status identified, and which may be applied in carrying out the method of the invention.

### Examples

### Material and methods

### Patients material

At the Trombotic Service Nijmegen sixteen patients on long term stable anticoagulation INR 2.5-4 age 50-70 were included. Patients gave a written informed consent after they received study information. Whole saliva was collected in the morning when patients did not drink more than 2 glasses of alcohol on the evening before and did not eat 1 hour before collection. In case of infections in the oral cave or a severe cold, patients were excluded. Blood was drawn at the same time. Healthy volunteers were matched for age and sex and were all from the Radboud University Nijmegen Medical Centre. Before saliva collection the mouth was rinsed with water and donors were allowed to chew on 25 cm2 sheet of parafilm (Parafilm® M) for 1 minute. Produced saliva was collected into a 50 ml tube and centrifuged at 10,000g for 10 minutes. Samples were divided in aliquots of 250 µl and stored at -80°C. Stimulated whole saliva collection was used because of its non-invasive approach instead of *e.g.* glandular saliva donation, and since stimulated whole saliva was found to have a large portion of peptides and proteins derived from the parotid glands.

### Proteomics

The SELDI-TOF-MS technology (Biorad, Veenendaal) consists of the protein chip array, mass spectrometer and data analysis software. Profiling was performed on three protein chip array surfaces (Q10, CM10 and IMAC-Cu). The binding/wash buffer for the anionic exchange arrays (Q10) contained 0.1% Triton X-100 with 0.1M Tris-HCl (pH 8) and that for the cationic exchange arrays (CM10) contained 0.1% Triton-X100 with 0.1 mM ammonium acetate pH 4.5. Both Q10 and CM10 chips were preincubated with binding buffer. The metal affinity-binding IMAC-Cu chips were loaded with 50 µl 0.1M copper sulphate by vigorous shaking for 10 min. After washing with water, the chip surface was neutralised using 150 µl sodium acetate buffer pH 4 followed by a short wash with water and pre-incubation with binding buffer (0.1M Tris-HCl, pH 7.4) containing 0.1% Triton X-100 and 100 mM NaCl. Saliva samples were diluted 1:100 in binding buffer (total volume of 100 µl) and applied at random and in duplicate to the chip and incubated for 1 h shaking on a mixer. Afterwards, spots were washed six times with 150 µl binding buffer for 10 min (three times with and three times without 0.1% Triton X-100). Before application of the matrix (Sinapinic acid (SPA; Bio-Rad) in 50% acetonitrile/0.1% trifluoroacetic acid), another shortwash with HPLC-gradewater was performed and the chips were airdried for 10 min. The matrix was applied twice (0.8 µl each time, 1 min apart) and the chips were airdried prior to reading on a ProteinChip Reader IIC instrument (Bio-Rad), using the following settings: detector sensitivity 9; detector voltage 2900; positions 20-80 were read with an increment of 10; 50 laser shots were collected on each position; two warming shots were fired at each position; lag time focus of 241 ns; laser intensity 180. Calibration was done with a 7-30 kDa proteins mixture. After baseline subtraction, peak labelling was performed with CiphergenExpress Software (version 3.0) for peaks with a signal-to-noise (S/N) ratio ≥3 in the *m*/*z* range from 1.5 to 100 kDa, and then normalized by total ion current.

### Statistics

Results are presented in scattergrams containing an average. Calculated p-values (Kruskal-Wallis/Mann Whitney U) were turned into Q-values correcting for a false discovery rate (R). Significant Q values (<0.05) were considered to be statistically significant. Some additional peaks with non significant Q values were also listed as these peaks showed to correlate with the INR.

### ELISA

Standard ELISA's for BNP-32, and thymosin β4 were performed.

### Preparation of protein fractions for identification

For the preparation of protein fractions SigmaPrep spin column kit (Sigma, Zwijndrecht, The Netherlands) and Q Hyper D beads (Biosepra, Paris, France) were used. 200 µl of Q Hyper D beads were pre-equilibrated in an end-over-end mixer 5 times using 100 mM Tris-HCl pH 9.0 0.1% OGP (equilibration buffer) for 5 minutes. 200 µl stimulated whole saliva obtained from a healthy volunteer, diluted in 300 µl equilibration buffer, was added to the beads, followed by an incubation period of 45 minutes. The columns were centrifuged at 2000 rpm in an Eppendorf centrifuge for 1 minute and the flowthrough was collected. Different fractions were sequentially eluted from the beads by a 10 minute incubation period with 400 µl of different elution buffers in an end-over-end mixer followed by centrifugation at 2000 rpm for 1 minute. The elution buffers were [1] 50 mM HEPES pH7.0 0.1% OGP for fraction pH7.0, [2] 100 mM NaAc pH5.0 0.1% OGP for fraction pH5.0, [3] 100 mM NaAc pH4.0 0.1% OGP for fraction pH4.0, [4] 100 mM sodium citrate pH3.0 0.1% OGP for fraction pH3.0, and [5] 33% isopropanol 17% acetonitril (ACN) 0.1% TFA for the organic fraction.

The fractions were analyzed using the NP20 protein chip array (1 µl) to evaluate relative abundance of the biomarkers compared to other proteins. The fraction containing the biomarkers was used for reversed phase chromatography or gel electrophoresis.

Further isolation of biomarkers was done with Poly-Bio Beads (Polymer Laboratories Inc, UK, 1412-1201) . 100 µl of Poly-Bio beads in an 1.5 ml eppendorf were pre-equilibrated in an end-over-end mixer 5 times using 800 µl 10 % acetonitrile, 0.1% TFA. The biomarker containing fraction was adjusted to a final concentration of 10% acetonitrile, 0.1 %TFA and incubated with the beads for 30 minutes at room temperature. The eppendorfs were centrifuged for 1 minute at 5000 rpm and the supernatant was transferred into a fresh tube. Different fractions were sequentially eluted from the beads by a 10 minute incubation time with 400 µl of different elution buffers in an end-over-end mixer followed by centrifugation at 5000 rpm for 1 minute. The elution buffers were [1] 10% acetonitrile, 0.1% TFA, [2] 20% acetonitrile, 0.1% TFA, [3] 30% acetonitrile, 0.1% TFA, [4] 40% acetonitrile, 0.1% TFA, [5] 50% acetonitrile, 0.1% TFA and [6] 70% acetonitrile, 0.1% TFA.

The fractions were analysed using the NP20 protein chip array (1 µl) to evaluate relative abundance of the biomarkers compared to other proteins. The fraction containing the biomarker, in this case the 50% acetonitrile fraction was used for identification.

### Gel electrophoresis

Fractions were dried in a SpeedVac. 20 µl of 0.7% NH₄OH was added. The sample was dried in a SpeedVac and resuspended in 10 µl 4x loading sample buffer and 10 µl dH₂O, followed by incubation at 70 °C for 20 minutes. These samples was loaded on a 18% bis-Tris non-reducing polyacrylamide gel (Invitrogen, Breda, The Netherlands). Gel electrophoresis was performed at 80V for 30 minutes, followed by electrophoresis at 120 V until the end of the gel was reached. After electrophoresis the gel was fixated in a 10% acetic acid 50% methanol solution for 15 minutes. After removing the fixating solution, the gel was stained overnight in a shaker with Coomassie blue (Invitrogen, Breda, The Netherlands). Staining was stopped by removal of staining solution and addition of water.

### Passive elution

To confirm that the excised bands from the gel were the correct proteins, passive elution was performed. Two plugs were excised using a 1mm diameter Harris micro-punch (Sigma, Germany) and placed into a 1.5 ml eppendorf tube. These plugs were sequentially transferred into [1] 200 µl of 50% methanol/10% acetic acid to remove staining for 30 minutes and [2] 100 µl acetonitrile. After removal of acetonitrile gelplugs were dried at 70°C for 3 minutes followed by extraction of proteins with 70 µl 50% formic acid, 25% acetonitrile, 15% isopropanol and 10% water under vigorous shaking for 60 minutes. The eluted proteins were analysed using the NP20 protein chip array (1 µl). If the correct band was excised, trypsin digestion was performed.

### Trypsin digestion

Bands were excised and sequentially transferred into [1] a 200 µl 50% methanol 10% acetic acid to remove staining for 30 minutes, [2] 200 µl of 50% ACN 100 mM NH₄HCO₃ for 30 minutes, and [3] 100 µl of ACN for 15 minutes ACN was removed and the gel was dried at 70 °C for 3 minutes. The dried gel pieces were rehydrated with 50 mM NH₄HCO₃ pH8.0 containing 10-20 ng/µl of modified trypsin (Promega, Madison, WI, USA), followed by incubation at room temperature for 16 hours.

### Protein identification by peptide mass fingerprinting using PBS-2C protein chip reader

Aliquots of 1 µl protease digest were spotted on a NP20 protein chip array and air-dried. 1 µl of 20% saturated α-Cyano-4-hydroxycinnamic (CHCA) in 50% ACN 0.5% TFA was applied to the spot, which subsequently was air-dried. The spectra were read in peptide mass range (0.5-5 kDa) with laser intensity 138 and lagtime 205 ns. Known trypsin autolysis peaks were used for internal calibration. The spectrum was manually collected. After analysis m/z values were submitted for a search of the NCBI database using the Profound search algorithm as the database mining tool.

### Protein identification by peptide fragmentation using a vMaldi-LTQ

Protein bands were cut from gel and were in-gel digested using trypsin as described elsewhere (Wilm, M., Shevchenko, A., Houthaeve, T., Breit, S. et al., Femtomole sequencing of proteins from polyacrylamide gels by nano-electrospray mass spectrometry. Nature 1996,379, 466-469). Resulting peptide mixtures were desalted & concentrated using STAGE tips (Rappsilber, J., Ishihama, Y., Mann, M., Stop and go extraction tips for matrix-assisted laser desorption/ionization, nanoelectrospray, and LC/MS sample pretreatment in proteomics. Anal. Chem. 2003, 75, 663-670) and were spotted onto a MALDI target plate using the dried droplet method. Briefly, 0.5ul sample was spotted on a stainless steel target plate and allowed to air dry. Next, 0.5ul CHCA matrix (2.5mg/ml CHCA, 50% acetonitrile, 0.05% trifluoroacetic acid) was added and left to crystallize under room conditions. Samples were analyzed using an intermediate vacuum pressure Matrix Assisted Laser Desorption Ionization Linear Ion Trap mass spectrometer (vMALDI LTQ; Thermo Scientific). The instrument was programmed to run 5 cycles on each sample. Each cycle consisted of a survey scan (full MS: 700-4000m/z) from which the top 10 most abundant ions were masses dependently selected for fragmentation analysis using collision induced dissociation (3m/z isolation width, 35% normalized collision energy, dynamic exclusion enabled). Raw data processing and Sequest database searches were performed using Bioworks software (Thermo scientific).

### Correlation of proteins to INR

The intensity of statherin, acidic salivary proline-rich protein 1/2 , and acidic salivary proline-rich protein 3/4 on a Q10 chip comprising salivary samples from patients on VKA therapy was correlated to the INR measured in said patients.

The intensity of S100A8 and α-haemoglobin on a CM10 chip comprising salivary samples from patients on VKA therapy was correlated to the INR measured in said patients.

The amount of BNP-32 (in ng/ml) was measured using standard ELISAs as described above in salivary samples from patients on VKA therapy. The amount of BNP-32 was correlated to the INR measured in said patients.

### Results

Analysis of profiling experiments yielded several differential expressed peaks when comparing controls with coumarin treated patients. The Q10 chip revealed 53 significant differences (P<0.05) using Mann-Whitney statistics. These values were corrected for the large number of variables (210) using the Q-test. After this transformation 29 significant Q-values remained (Q-value <0.05). Only the best values were further analyzed. As a validation of the system also a random classifier was added to see how many significant peaks were falsely discovered. After randomization 9 significant differences were detected. Correction with the Q-test gave no significant values indicating that the 29 significant proteins are true differences.

Within these significantly differentially expressed proteins and a few interesting non-significantly differentially expressed proteins a new classifier was used to look at INR levels and intensity of the peaks. All patients that were included were supposed to have an INR within the therapeutic range (2.5-4). However, two patients had an INR within the range 1-2, four patients within the range 2-3 and ten patients within the range 3-4.

**Table 1. Potential biomarkers identified on a Q10 chip based on Q values**

| m/z | Peptide/protein Description based on m/z values or otherwise mentioned. | Accession number | Molecular weight (Da) | Pi | p-value | q-value |
|---|---|---|---|---|---|---|
| 5409 | Statherin* | P02808 | 5220 | 6.25 | 0.0003 | 0.0202 |
| 5432 | Statherin* | P02808 | 5220 | 6.25 | 0.0004 | 0.0202 |
| 26618 | Unknown | | | | 0.0006 | 0.0202 |
| 2706 | BNP(4-29)** | P16860 | 2704 | 9.5 | 0.0007 | 0.0202 |
| | Motilin | P12872 | 2699 | 8.5 | | |
| 4176 | Glucagon-like peptide 1 | P01275 | 4170 | 5.05 | 0.0007 | 0.0202 |
| | Pancreatic hormone | P01298 | 4183 | 6.25 | | |
| 5656 | Beta-defensin 111 | Q30KQ9 | 5655 | 8.88 | 0.0009 | 0.0202 |
| | ATP synthase subunit epsilon, mitochondrial | P56381 | 5649 | 9.93 | | |
| 16541 | Unknown | | | | 0.0009 | 0.0202 |
| 3245 | BNP(2-31)** | P16860 | 3242 | 10.96 | 0.0011 | 0.0208 |
| | Ghrelin-28 | Q9UBU3 | 3245 | 11.07 | | |
| 5632 | Thymopoietin | P42166 | 5638 | 7.16 | 0.0012 | 0.0208 |
| | Osteocrin | P61366 | 5621 | 11.8 | | |
| 1968.88 | Neuropeptide AF | O15130 | 1979 | 6.04 | 0.0014 | 0.0215 |
| 5612 | Osteocrin | P61366 | 5621 | 11.8 | 0.0018 | 0.0235 |
| 16353 | Unknown | | | | 0.0018 | 0.0235 |
| 19668 | Unknown | | | | 0.0020 | 0.0248 |
| 2623 | ABri/ADan amyloid peptide | Q9Y287 | 2630 | 5.5 | 0.0023 | 0.0251 |
| 4932 | Thymosin beta-4-like protein 3 | A8MW06 | 4932 | 5.03 | 0.0027 | 0.0251 |
| | Thymosin beta-4** | P62328 | 4921 | 5.03 | | |
| | Thymosin beta-4-like protein 1 | Q08EQ4 | 4940 | 5.03 | | |
| 5388 | Statherin* | P02808 | 5220 | 6.25 | 0.0027 | 0.0251 |
| 2468 | Proadrenomedullin N-20 terminal peptide | P35318 | 2462 | 11 | 0.0030 | 0.0251 |
| | Relaxin-3 A chain | Q8WXF3 | 2464 | 6.04 | | |
| 5588 | Epiregulin. | 014944 | 5573 | 6.01 | 0.0030 | 0.0251 |
| | Peptide P-H. (PRP-1) | P04280 | 5574 | 11.71 | | |
| 20877 | Unknown | | | | 0.0030 | 0.0251 |
| 2695 | Motilin. | P12872 | 2699 | 8.5 | 0.0034 | 0.0258 |
| | BNP(4-29)** | P16860 | 2704 | 9.5 | | |
| | Humanin | Q8IVG9 | 2687 | 9.5 | | |
| 3167 | BNP(1-30)** | P16860 | 3173 | 10.32 | 0.0034 | 0.0258 |
| 4200 | Apelin-36 | Q9ULZ1 | 4196 | 12.8 | 0.0050 | 0.0342 |
| 16201 | Unknown | | | | 0.0050 | 0.0342 |
| 1693 | Neurotensin | P30990 | 1690 | 9.68 | 0.0064 | 0.0418 |
| | Calcitermin | P80511 | 1689 | 8.49 | | |
| | Tryptase gamma light chain | Q9NRR2 | 1699 | 8.24 | | |
| 22375 | Unknown | | | | 0.0081 | 0.0489 |
| 27615 | Unknown | | | | 0.0081 | 0.0489 |
| 3294 | Glucagon-like peptide 1(7-36) | P01275 | 3299 | 5.53 | 0.0091 | 0.0492 |
| | BNP(3-32)** | P16860 | 3282 | 10.93 | | |
| | Apelin-28 | Q9ULZ1 | 3302 | 12.69 | | |
| 11169 | salivary acidic proline-rich phosphoprotein ¾ * | P02810 | 11019 | 4.14 | 0.0091 | 0.0492 |
| 63241 | Unknown | | | | 0.0091 | 0.0492 |
| 15515 | salivary acidic proline-rich phosphoprotein ½* | P02810 | 15372 | 4.63 | 0.0114 | 0.0528 |

**Table 2. Potential biomarkers identified on a CM10 chip based on Q values**

| m/z | Peptide/protein Description | Accession number | Molecular weight (Da) | Pi | p-value | q-value |
|---|---|---|---|---|---|---|
| 3246 | BNP(2-31)** | P16860 | 3242 | 10.96 | 0.0008 | 0.0275 |
| | Ghrelin-28 | Q9UBU3 | 3245 | 11.07 | | |
| 2831 | BNP(3-29)** | P16860 | 2832 | 9.84 | 0.0009 | 0.0275 |
| 4819 | Unknown | | | | 0.0012 | 0.0275 |
| 2626 | ABri/ADan amyloid peptide | Q9Y287 | 2630 | 5.5 | 0.0014 | 0.0275 |
| 3167 | BNP(1-30)** | P16860 | 3173 | 10.31 | 0.0016 | 0.0275 |
| 3295 | Glucagon-like peptide 1(7-36 | P01275 | 3299 | 5.53 | 0.0018 | 0.0275 |
| | Apelin-28 | Q9ULZ1 | 3302 | 12.69 | | |
| 4903 | Thymosin beta-4-like protein 6 | A9Z1Y9 | 4911 | 5.03 | 0.0020 | 0.0275 |
| | Cytochrome c oxidase polypeptide 8A | P10176 | 4894 | 6.07 | | |
| | Thymosin beta-10 | P63313 | 4894 | 5.30 | | |
| | Thymosin beta-4-like protein 2 | Q2KHM7 | 4903 | 5.03 | | |
| 4935 | Thymosin beta-4-like protein 3 | A8MW06 | 4932 | 5.03 | 0.0030 | 0.0277 |
| | Thymosin beta-4-like protein 1 | Q08EQ4 | 4940 | 5.03 | | |
| 2469 | Proadrenomedullin N-20 terminal peptide | P35318 | 2462 | 11 | 0.0034 | 0.0277 |
| | Relaxin-3 A chain | Q8WXF3 | 2464 | 6.04 | | |
| 11895 | Natriuretic peptides B** | P16860 | 11906 | 10.13 | 0.0034 | 0.0277 |
| | Caspase-3 subunit p12 | P42574 | 11896 | 6.37 | | |
| | Protein CASC2, isoform 3 | Q6XLA1 | 11902 | 8.51 | | |
| 4057 | Peptide YY(3-36 | P10082 | 4051 | 6.76 | 0.0039 | 0.0277 |
| | Histatin-3 | P15516 | 4062 | 9.99 | | |
| | Putative DNA-binding protein inhibitor ID-2B | Q14602 | 4055 | 10.26 | | |
| 12164 | Bone morphogenetic protein 10 | O95393 | 12160 | 8.66 | 0.0039 | 0.0277 |
| | Transmembrane protein 14D | A8MWL7 | 12178 | 9.58 | | |
| | | | | | | |
| 5016 | Somatomedin-B. | P04004 | 5012 | 4.50 | 0.0044 | 0.0277 |
| | Endokinin-A | Q86UU9 | 5055 | 3.99 | | |
| 4007 | Unknown | | | | 0.0050 | 0.0277 |
| 4431 | CART(1-39) | Q16568 | 4424 | 4.48 | 0.0050 | 0.0277 |
| 8192 | Unknown | | | | 0.0050 | 0.0277 |
| 9859 | Lymphocyte antigen 6H | O94772 | 9860 | 6.26 | 0.0050 | 0.0277 |
| | Cyclin-dependent kinases regulatory subunit 2 | P33552 | 9860 | 8.07 | | |
| | Pro-MCH-like protein 2 | Q9BQD1 | 9856 | 8.66 | | |
| 1926 | Unknown | | | | 0.0063 | 0.0300 |
| 5137 | Complement C3g fragment | P01024 | 5137 | 3.97 | 0.0063 | 0.0300 |
| | Transmembrane protein 167A | Q8TBQ9 | 5143 | 9.41 | | |
| 8410 | Unknown | | | | 0.0064 | 0.0300 |
| 2882 | BNP(5-31)** | P16860 | 2885 | 10.85 | 0.0072 | 0.0307 |
| | P-beta | Q9H300 | 2876 | 10.93 | | |
| 5095 | NB thymosin beta | Q99406 | 5098 | 5.30 | 0.0072 | 0.0307 |
| | Adrenomedullin-2 | Q7Z4H4 | 5104 | 7.79 | | |
| 38997 | Unknown | | | | 0.0102 | 0.0417 |
| 11378 | Unknown | | | | 0.0128 | 0.0462 |
| 11729 | Unknown | | | | 0.0128 | 0.0462 |
| 97923 | Unknown | | | | 0.0142 | 0.0497 |
| 5693 | Beta-defensin 131 | P59861 | 5702 | 6.07 | 0.0159 | 0.0499 |
| 15804 | Unknown | | | | 0.0159 | 0.0499 |
| 28621 | Unknown | | | | 0.0159 | 0.0499 |
| 10841 | S100A8* | P05109 | 10835 | 6.51 | 0.0297 | 0.0582 |
| 12681 | S100A9* (de et al., 2008) | P06702 | 12688 | 5.71 | 0.0690 | 0.0877 |
| 15111 | α-haemoglobin (de et al., 2008;Woong-Shick et al., 2005) | P69905 | 15126 | 8.74 | 0.1665 | 0.1582 |

**Table 3. Potential biomarkers identified on an IMAC chip based on Q values**

| m/z | Peptide/protein Description | Accession number | Molecular weight (Da) | Pi | p-value | q-value |
|---|---|---|---|---|---|---|
| 18122 | Unknown | | | | 0.0002 | 0.0204 |
| 55881 | Unknown | | | | 0.0010 | 0.0407 |
| 57792 | Unknown | | | | 0.0010 | 0.0407 |

| | | | | | | |
|---|---|---|---|---|---|---|
| * Identified via peptide mapping or sequenced by high resolution ms-instruments ** Measured using an EIA or ELISA | | | | | | |

de SD, Fillet M, Ribbens C, Maree R, Meuwis M A, Lutteri L, Chapelle J P, Wehenkel L, Louis E, Merville M P and Malaise M (2008) Monomeric Calgranulins Measured by SELDI-TOF Mass Spectrometry and Calprotectin Measured by ELISA As Biomarkers in Arthritis. Clin Chem 54:1066-1075 .
Woong-Shick A, Sung-Pil P, Su-Mi B, Joon-Mo L, Sung-Eun N, Gye-Hyun N, Young-Lae C, Ho-Sun C, Heung-Jae J, Chong-Kook K, Young-Wan K, Byoung-Don H and Hyun-Sun J (2005) Identification of Hemoglobin-Alpha and -Beta Subunits As Potential Serum Biomarkers for the Diagnosis and Prognosis of Ovarian Cancer. Cancer Sci 96:197-201.

### Correlation between level of proteins and INR

The amount of BNP-32 (ng/ml) measured with EIA was negatively correlated to the INR measured in citrated plasma (r²=0.6128; P<0.05).

The intensity of S100A8 on a CM10 chip was negatively correlated to the INR measured in citrated plasma (r²=0.514; P<0.05).

The intensity of acidic salivary proline-rich protein 1/2 on a Q10 chip and the INR measured in citrated plasma were negatively correlated (r²=0.3501; P<0.05).

The intensity of statherin on a Q10 chip and the INR measured in citrated plasma were negatively correlated (r² =0.2687; P<0.05).

## Claims

1. A non-invasive method for determining the blood coagulation status of a subject comprising the steps of:
a) providing a sample of saliva of said subject;
b) determining the level of one or more biomarkers, in said sample of saliva;
c) correlating the amount of said one or more biomarkers to the blood coagulation status, wherein one of said biomarkers is statherin.

2. A method according to claim 1, wherein the blood coagulation status is determined by the international normalized ratio (INR).

3. A method according to any one of the preceding claims, wherein the amount of said one or more biomarkers is determined using an enzyme immunoassay or mass spectrometry.

4. A method according to any of the preceding claims, wherein the blood coagulation status is related to the intake of anticoagulant or procoagulant drugs.

5. A method according to any of the preceding claims, wherein the method is performed in a non-invasive device for *in vitro* analysis.

6. A method according to any of the preceding claims, wherein the subject is undergoing Vitamin K antagonist (VKA) therapy, or other anticoagulant or procoagulant therapy.

7. A method according to any of the preceding claims, wherein the blood coagulation status is related to a blood coagulation disease or disorder, wherein the blood coagulation disease or disorder preferably is an acute thrombotic situation, such as acute myocardial infarction or pulmonary embolism or an acute bleeding situation.

## Patentansprüche

1. Nicht-invasives Verfahren zur Bestimmung des Blutgerinnungsstatus einer Person, welches die Schritte umfasst:
a) Bereitstellen einer Speichelprobe der besagten Person;
b) Bestimmen des Spiegels von einem oder mehreren Biomarker(n) in besagter Speichelprobe;
c) Korrelieren der Menge von besagtem/besagten einen oder mehreren Biomarker(n) mit dem Blutgerinnungsstatus, wobei einer der besagten Biomarker Statherin ist.

2. Verfahren nach Anspruch 1, wobei der Blutgerinnungsstatus durch die International Normalized Ratio (INR) bestimmt wird.

3. Verfahren nach einem der vorangehenden Ansprüche, wobei die Menge von besagtem/besagten einen oder mehreren Biomarker(n) durch Verwenden eines Enzymimmunoassay oder Massenspektrometrie bestimmt wird.

4. Verfahren nach einem der vorangehenden Ansprüche, wobei der Blutgerinnungsstatus mit der Einnahme von anticoagulierenden oder procoagulierenden Medikamenten zusammenhängt.

5. Verfahren nach einem der vorangehenden Ansprüche, wobei das Verfahren in einer nicht-invasiven Vorrichtung zur *in vitro-*Analyse durchgeführt wird.

6. Verfahren nach einem der vorangehenden Ansprüche, wobei sich die Person einer Vitamin-K-Antagonist (VKA)-Therapie unterzieht, oder einer anderen anticoagulierenden oder procoagulierenden Therapie.

7. Verfahren nach einem der vorangehenden Ansprüche, wobei der Blutgerinnungsstatus mit einer Blutgerinnungserkrankung oder -störung zusammenhängt, wobei die Blutgerinnungserkrankung oder -störung bevorzugt eine akute thrombotische Situation ist, zum Beispiel ein akuter Myokardinfarkt oder eine pulmonale Embolie oder eine akute Blutung.

## Revendications

1. Méthode non invasive de détermination du statut de coagulation sanguine d'un sujet comprenant les étapes consistant à :
a) fournir un échantillon de salive dudit sujet ;
b) déterminer le niveau d'un ou plusieurs biomarqueurs dans ledit échantillon de salive ;
c) corréler la quantité desdits un ou plusieurs biomarqueurs au statut de coagulation sanguine, dans lequel l'un desdits biomarqueurs est la stathérine.

2. Procédé selon la revendication 1, dans lequel l'état de coagulation sanguine est déterminé par le rapport normalisé international (INR).

3. Procédé selon l'une quelconque des revendications précédentes, dans lequel la quantité dudit un ou de plusieurs biomarqueurs est déterminée en utilisant un dosage immuno-enzymatique ou une spectrométrie de masse.

4. Procédé selon l'une quelconque des revendications précédentes, dans lequel le statut de coagulation sanguine est lié à la prise de médicaments anticoagulants ou procoagulants.

5. Procédé selon l'une quelconque des revendications précédentes, dans lequel le procédé est effectué dans un dispositif non invasif pour une analyse *in vitro.*

6. Procédé selon l'une quelconque des revendications précédentes, dans lequel le sujet est soumis à un traitement par antagoniste de vitamine K (AVK) ou à un autre traitement par anticoagulants ou procoagulants.

7. Procédé selon l'une quelconque des revendications précédentes, dans lequel le statut de coagulation sanguine est lié à une maladie ou un trouble de la coagulation sanguine, dans lequel la maladie ou le trouble de la coagulation sanguine est de préférence une situation thrombotique aiguë, comme un infarctus aigu du myocarde ou une embolie pulmonaire, ou une situation de saignement aigu.
